Europäisches Patentamt

European Patent Office

Office européen des brevets

(19)

(11) Veröffentlichungsnummer : **0 123 906**
B1

(12)    # EUROPÄISCHE PATENTSCHRIFT

(45) Veröffentlichungstag der Patentschrift :
06.05.87

(51) Int. Cl.⁴ : **C 07 C121/60**

(21) Anmeldenummer : 84103401.0

(22) Anmeldetag : 28.03.84

(54) **Verfahren zur Herstellung von Stilben-dicarbonitrilen.**

(30) Priorität : 02.04.83 DE 3312164

(43) Veröffentlichungstag der Anmeldung :
07.11.84 Patentblatt 84/45

(45) Bekanntmachung des Hinweises auf die Patenterteilung : 06.05.87 Patentblatt 87/19

(84) Benannte Vertragsstaaten :
CH DE FR GB IT LI

(56) Entgegenhaltungen :
DE-A- 3 119 992
THE JOURNAL OF ORGANIC CHEMISTRY, Band 47, Nr. 12, 4. Juni 1982, Seiten 2471-2474, American Chemical Society, US; G.P. SOLLOTT: "Conversion of 2,4,6-trinitrobenzyl chloride to 2,2',4,4',6,6' -hexanitrostilbene by nitrogen bases"
BULLETIN DE LA SOCIETE CHIMIQUE DE FRANCE, Nr.5, 1973, Seiten 1804-1805, Artikel Nr. 309, Masson et Cie., Paris, FR; C. GANSSER u.a.: "Sur l'obtention de l'ortho-nitrophényl-1 ortho-nitro para-chlorophényl-2 éthylène (note de laboratoire)"
SYNTHESIS - INTERNATIONAL JOURNAL OF METHODS IN SYNTHETIC ORGANIC CHEMISTRY, Nr. 5, Mai 1983, Seiten 341-368, K.B. BECKER: "Synthesis of stilbenes"

(73) Patentinhaber : **HOECHST AKTIENGESELLSCHAFT**
**Postfach 80 03 20**
**D-6230 Frankfurt am Main 80 (DE)**

(72) Erfinder : **Heiss, Lorenz, Dr.**
**Stormstrasse 39**
**D-6238 Hofheim am Taunus (DE)**

## Beschreibung

Eine technisch verwertbare Synthese von Stilben-dicarbonitril ist nicht bekannt. Toland et al. beschreiben in dem US-Patent 2 688 631 eine dehydrierende Dimerisierung von p-Tolunitril mit Schwefel bei 260-300 °C mit sehr geringen Ausbeuten. Das von P. Walden im Ber. D. Chem. Ges. 23 (1890), 1959 beschriebene Verfahren für die Herstellung von Dinitrostilben aus p-Nitrobenzylchlorid in alkoholischer Kalilauge führt beim p-Cyanbenzylchlorid nicht zur Stilbenverbindung.

Überraschend wurde nun gefunden, daß man mit ausgezeichneten Ausbeuten und hoher Reinheit zu Stilben-dicarbonitrilen gelangen kann, wenn man das leicht zugängliche o- oder p-Cyanbenzylchlorid in aprotischen Lösungsmitteln mit einem Überschuß von Alkalihydroxypulver bei etwa Raumtemperatur unter Rühren reagieren läßt. Vorteilhaft ist dabei eine inerte Stickstoffatmosphäre und Feuchtigkeitsausschluß sowie möglichst feinpulvriges, trockenes Alkalihydroxyd. Als aprotisches polares Lösungsmittel kommt vor allem Dimethylformamid in Frage. Die Reaktion wird bei Raumtemperatur solange durchgeführt, bis alles organische Chlor reagiert hat. Die Reaktionszeit beträgt im allgemeinen 6 bis 12 Stunden. Nach Beendigung der Reaktion säuert man an, vorzugsweise mit wasserfreien organischen Säuren wie etwa Eisessig. Man saugt vom Reaktionsgemisch ab, wäscht mit Lösungsmittel und dann mit Wasser nach, bis keine Chloridionen mehr nachweisbar sind. Man erhält Stilben-4,4'-dicarbonitril bzw. Stilben-2,2'-dicarbonitril in weißen Kristallen in Ausbeuten über 80 %.

Diese Stilbenverbindungen können zur Stilbendicarbonsäure verseift werden, woraus Bisbenzoxazolyl-stilbene hergestellt werden können, die als optische Aufheller Verwendung finden.

## Beispiel 1

in 100 g Dimethylformamid werden 24 g (0,6 Mol) Natriumhydroxydpulver unter Stickstoffeinleitung dispergiert und bei 20 °C 30,4 g (0,2 Mol) p-Cyanbenzylchlorid zugefügt. Man rührt ca. 6 — 12 Stunden bis alles organisch gebundene Chlor reagiert hat, dann werden 30 g (0,5 Mol) Eisessig zugesetzt. Nach dem Abkühlen auf 0 °C, saugt man ab, wäscht mit Dimethylformamid und dann mit Wasser nach, bis im Waschwasser keine Chloridionen nachweisbar sind. Nach dem Trocknen erhält man in weißen Kristallen 18,9 g Stilben-4,4'-dicarbonitril.

Ausbeute : 82 %,     Schmelzpunkt : 288 °C

C 83,5 % berechnet ;     83,3 % gefunden
H  4,3 % berechnet ;      4,3 % gefunden
N 12,2 % berechnet ;     12,1 % gefunden

## Beispiel 2

In 70 g Dimethylformamid werden 30,4 g (0,2 Mol) o-Cyanbenzylchlorid gelöst und im Stickstoffstrom 24 g (0,6 Mol) Natriumhydroxydpulver eingetragen. Man rührt bei 20 °C ca. 6 — 10 Stunden nach bis alles organisch gebundene Chlor reagiert hat, tropft 70 g Methanol zu, saugt bei 0 °C ab, wäscht mit Methanol, dann mit Wasser nach bis im Waschwasser keine Chloridionen nachweisbar sind. Nach dem Trocknen erhält man in weißen Kristallen 17,8 g Stilben-2,2'-dicarbonitril.

Ausbeute : 77,5 %,     Schmelzpunkt : 187-189 °C

C 83,5 % berechnet ;     83,1 % gefunden
H  4,3 % berechnet ;      4,3 % gefunden
N 12,2 % berechnet ;     12,0 % gefunden

## Patentanspruch

Verfahren zur Herstellung von Stilben-dicarbonitrilen, dadurch gekennzeichnet, daß man o- oder p-Cyanbenzyl-chlorid im polaren aprotischen Lösungsmittel mit einem Überschuß von Alkalihydroxydpulver dimerisiert unter Abspaltung von Chlorwasserstoff.

## Claim

A process for preparing stilbenedicarbonitriles which comprises dimerizing o- or p-cyanobenzyl chloride in a polar aprotic solvent in the presence of an excess of alkali metal hydroxide powder with loss of hydrogen chloride.

## Revendication

Procédé de préparation de stilbène-dicarbonitriles, procédé caractérisé en ce qu'on dimérise, avec enlèvement de chlorure d'hydrogène, le chlorure d'o-cyano-benzyle ou le chlorure de p-cyano-benzyle. dans un solvant aprotique polaire, au moyen d'un excès d'un hydroxyde de métal alcalin en poudre.